# EUROPÄISCHE PATENTSCHRIFT

(11) **EP 2 960 324 B1**
(45) Veröffentlichungstag und Bekanntmachung des Hinweises auf die Patenterteilung: **17.03.2021**
(21) Anmeldenummer: 15173480.3
(22) Anmeldetag: 23.06.2015
(51) Int. Cl.: C12M 1/107, C12M 1/00, C12M 1/02, C12M 1/12, C12M 3/00

(54) **VERFAHREN ZUR HERSTELLUNG VON BIOGAS IN EINEM FERMENTER UND VORRICHTUNG ZUR DURCHFÜHRUNG DES VERFAHRENS**
METHOD FOR THE PREPARATION OF BIOGAS IN A FERMENTER AND DEVICE FOR CARRYING OUT THE METHOD
PROCEDE DE PRODUCTION DE BIOGAZ DANS UN FERMENTEUR ET DISPOSITIF D'EXECUTION DU PROCEDE

(30) Priorität: 23.06.2014 DE 102014008958
(43) Veröffentlichungstag der Anmeldung: 30.12.2015
(73) Patentinhaber: Broekhuijsen, Andries Johan, 48249 Dülmen (DE); Broekhuijsen, Johan Jakob, 48240 Dülmen (DE)
(72) Erfinder: Broekhuijsen, Andries Johan, 48249 Dülmen (DE); Broekhuijsen, Johan Jakob, 48240 Dülmen (DE)
(74) Vertreter: Tarvenkorn, Oliver

(56) Entgegenhaltungen:
- EP-A1- 2 336 292
- CA-A1- 2 731 716
- CN-A- 102 936 566
- DE-A1- 3 151 187
- DE-A1-102008 038 065
- DE-A1-102009 007 902

## Beschreibung

Die Erfindung betrifft ein Verfahren zur Herstellung von Biogas in einem Fermenter, mit den Merkmalen des Oberbegriffs des Anspruchs 1. Zudem betrifft die Erfindung eine Vorrichtung zur Durchführung des Verfahrens mit den Merkmalen des Oberbegriffs des Anspruchs 9.

Verfahren der eingangs genannten Art sind aus dem Stand der Technik bekannt und werden in Biogasanlagen durchgeführt, deren wesentlicher Bestandteil ein Fermenter ist, in dem Biogas, d. h. ein Gemisch aus vorwiegend Methan und Kohlenstoffdioxid, in einer anaeroben Vergärung aus Biomasse gewonnen wird. Als Gärsubstrate werden gewöhnlich unter anderem Gülle, Klärschlamm, Grünabfälle, Getreide, Maissilage, Zuckerrüben und dergleichen eingesetzt. Holz gehört aufgrund seiner festen Substanz und langkettigen Zellulose-Strukturen bisher zu den weniger bevorzugten Substratkomponenten .

Der Fermenter kann gemäß dem Stand der Technik verschiedene Bauformen aufweisen. Er kann zylindrisch aufrecht oder quadratisch liegend sein. Herkömmliche Fermenter sind häufig zylindrisch aufrechtstehende, isolierte Behälter, in denen das Substrat ständig gerührt wird und in denen an jeder Stelle die gleiche Temperatur und die gleiche Konzentration der Umsetzungsstoffe in dem Substrat vorherrschen. Auch werden liegende Fermenter mit quadratischer Form eingesetzt, in denen das Substrat ebenfalls gerührt wird und an jeder Stelle die gleiche Temperatur vorherrscht.

Die erste Phase des Biogasgewinnungsprozesses, die Hydrolyse, ist eine biochemische Umsetzung, in der polymere Substanzen unter Wassereinlagerung in ihre Einzelbausteine gespalten werden. Die anschließende Vergärung vollzieht sich in mehreren Phasen, an denen unterschiedliche Bakteriengruppen beteiligt sind. Die erste bakterielle Phase stellt die sogenannte Acidogenese dar. In dieser Phase werden die zuvor in der Hydrolyse gebildeten Monomere von Bakterien aufgenommen und zu unterschiedlichen organischen Säuren und Alkoholen weiter vergoren. In einer sich anschließenden Phase, in der sogenannten Acetogenese, werden bestimmte Säuren von acetogenen Bakterien zu Essigsäure vergoren. In der finalen Phase, in der Methanogenese, wird die Essigsäure schließlich zu Methan und Kohlenstoffdioxid umgesetzt. Das hierbei erzeugte Biogas wird gereinigt und anschließend in der Regel in einem Blockheizkraftwerk in Strom und Wärme umgewandelt oder nach einer entsprechenden Gasaufbereitung ins Erdgasnetz eingespeist.

Bei der Biogasvergärung wird unter anderem zwischen mesophilen Prozessstufen, die bei einer Temperatur zwischen ca.25° und 40° C arbeiten, und thermophilen Prozessstufen, die bei einer Temperatur zwischen 40° C und 55 ° C arbeiten, unterschieden. Fermenter mit thermophilen Prozessstufen haben in der Regel innerhalb einer bestimmten Zeiteinheit eine höhere Biogasausbeute. Die thermophile Umsetzung ist im Vergleich schneller als die mesophile Umsetzung. Andererseits ist der thermophile Vergärungsprozess im Vergleich zur mesophilen Umsetzung weniger stabil und bei nicht optimal isolierten Fermentern der Wärmebedarf für einen stabilen thermophilen Prozessablauf höher. Somit weisen Fermenter mit mesophiler und thermophiler Betriebsweise unterschiedliche Vorteile auf. Wie aus der Natur bekannt ist, werden langkettige energetische Strukturen niemals in nur einem einzelnen Prozessschritt umgesetzt. Jedoch bleibt festzustellen, dass die Fermenter nach aktuellem Stand der Technik nur über eine einzelne Prozessstufe verfügen. Bei der in der DE 10 2008 038 065 A1 beschrieben Anlage zur Biogasgewinnung findet die gesamte biochemische Zersetzung, also die Hydrolyse, und die biologische mikrobakterielle Zersetzung in aktiv gerührten Fermentern statt. Dieser gleichzeitige Ablauf bringt erhebliche Nachteile bezüglich des gesamten Zersetzungsprozesses mit sich.

Außerdem durchströmt das Substrat im Gegenstromprinzip mindestens zwei Reaktorbehälter, welche mit zwei Substrat-Förderschneckentransportvorrichtungen betrieben werden. Dies erhöht die Wartungs- und Reparaturempfindlichkeit erheblich.

DE 31 51 187 A1 offenbart einen Gärbehälter mit thermisch getrennten Zonen. Bei dem Gärbehälter handelt es sich um ein stehendes Reaktorgefäß, mit dem keine natürliche, lediglich auf Verdrängung beruhende Förderung erreicht wird. Die Förderung erfolgt mit angetriebenen Elementen wie Pumpen, die aufgrund der damit in der Flüssigphase verbundenen Scherspannungen für das Bakterienwachstum nachteilig sind.

Gleiches gilt für die in DE 10 2008 038065 A1 offenbarte Vorrichtung, bei der die Reaktorgefäße stehend angeordnet sind und wiederum durch Rohrleitungen über Pumpen befüllt und entleert werden.

CA 2 731 716 A1 offenbart einen horizontal liegenden anaeroben Fermenter zur Herstellung von Biogas sowie ein Verfahren zur Biogaserzeugung. Der Fermenter verfügt über äußere Heizsysteme mit mehr als zwei unterschiedliche Temperaturzonen, die unabhängig voneinander arbeiten und geregelt werden. Die unterschiedlich beheizbaren Segmente des liegenden Bioreaktors werden jedoch temperaturkonstant gehalten, wobei die Zersetzung ausschließlich im thermophilen Bereich vorgesehen ist.

Es ist deshalb Aufgabe der vorliegenden Erfindung, ein Verfahren der eingangs genannten Art zur Verfügung zu stellen, das die Vorteile einer thermophilen Betriebsweise mit den Vorteilen einer mesophilen Betriebsweise in einem Fermenter vereint.

Zur Lösung dieser Aufgabe schlägt die Erfindung ein Verfahren mit den Merkmalen des Anspruchs 1 vor, bei dem das Substrat zwischen den beiden Enden des Fermenters hintereinander angeordnete Fermentersegmente oder -zonen durchströmt, in denen das Substrat unterschiedlich temperiert wird, also so erwärmt und/oder abgekühlt wird, dass für die in dem jeweiligen Längenabschnitt oder Segment ablaufenden Prozesse gute Reaktionsbedingungen bestehen.

Ein Fermenter im Sinne der vorliegenden Erfindung umfasst wenigstens ein einzelnes Strömungsrohr, das abschnittsweise temperierbar ist.

Erfindungsgemäß ist insbesondere vorgesehen, dass das Strömungsrohr abschnittsweise in Segmente unterteilt ist. Zwar können auch mehrere solcher segmentierten Strömungsrohre parallel betrieben werden, jedoch ist wichtig, dass jedes Strömungsrohr für sich über die Länge gesehen verschiedene Abschnitte umfasst, die einzeln oder gruppenweise temperierbar sind.

Das erfindungsgemäße Verfahren hat zunächst den Vorteil, dass während der Durchströmung des Substrats durch den Fermenter in einem Durchlauf sowohl mit einem mesophilen als auch mit einem thermophilen Temperaturbereich gearbeitet werden kann. Die einzelnen Fermenterzonen oder -segmente, von denen vorzugsweise mindestens zwei vorhanden sind, zeichnen sich unter anderem dadurch aus, dass sie unterschiedliche Temperaturzonen widerspiegeln.

Die hintereinander angeordneten Fermenterzonen oder - segmente entsprechen somit Prozessstufen mit entsprechenden Prozesstemperaturen. Ist beispielsweise im Rahmen einer thermophilen Prozessstufe der Wärmebedarf höher, wird in einem hierfür vorgesehenen Fermentersegment das Substrat während seiner Durchströmung durch den Fermenter entweder weniger stark gekühlt oder je nach Bedarf erwärmt, z.B. mittels einer aktiven Prozess- und Temperatursteuerung. Auf eine aktive Durchmischung des Substrats kann im Rahmen des erfindungsgemäßen Verfahrens verzichtet werden.

Eine grundlegende Idee der Erfindung ist es, die natürlichen biochemischen Zersetzungs-prozesse in ihrer Entwicklung und ihrem Wachstum nachzuempfinden und die einzelnen Phasen der mikrobakteriellen Prozesse durch gezielte Maßnahmen zu bremsen oder zu beschleunigen, insbesondere um durch geeignete Temperatur die Teilprozesse bei der Vergärung zu fördern.

Um also entlang der Fermenterlänge sowohl eine mesophile als auch eine thermophile Betriebsweise zu realisieren, wird von außen und/oder innerhalb des Fermenters segment- oder streckenweise die Temperatur variiert.

Die gesamte biochemische Zersetzung findet erfindungsgemäß bevorzugt in genau einem liegenden Strömungsrohr statt, das den Fermenter bildet. Der Fermenter bzw. sein Strömungsrohr ist vereinfacht vorzustellen wie ein längliches Gefäß mit zylindrischem Querschnitt oder ähnlicher Form, z.B. vieleckig oder konisch größer werdender Form.

Entscheidendes Merkmal der Erfindung ist, dass es sich um einen kontinuierlich ablaufenden Prozess mit Eingang, Durchlauf mehrerer Zersetzungsphasen und Ausgang handelt. Erfindungsgemäß ist vorgesehen, dass das Substrat innerhalb des kontinuierlich verlaufenden Prozesses mehrere mikrobakterielle Phasen durchläuft. Die Phasen beinhalten sowohl mesophile als auch thermophile bakteriellen Temperaturbereiche. Die unterschiedlichen Temperaturbereiche, die Resultat natürlicher mikrobakteriellen Zersetzungsprozesse sind, werden durch das erfindungsgemäße Verfahren gezielt unterstützt.

Die in den marktüblichen Fermentern vorgesehene mechanische Durchmischung, die zum Beispiel mittels Paddel-Rührwerken erfolgt, erzeugt lokal hohe Strömungsgeschwindigkeiten im Substrat, und die damit verbundenen Scherspannungen haben negative Auswirkungen auf die natürlichen biochemischen Zersetzungsprozesse. Entgegen der üblichen Praxis mit gerührten Fermentern werden mit dem erfindungsgemäßen Verfahren, das eine starke Durchmischung oder sogar jede Form von motorisch angetriebenen Mischern vermeidet, die biochemischen Zersetzungsprozesse sehr viel optimaler und natürlicher unterstützt.

Demnach ist nach der Erfindung vorzugsweise für die Durchmischung des Substrates eine gleitende Bewegung vorgesehen, ähnlich dem Prinzip der Peristaltik, welche insbesondere durch statische, strömungsleitende Elemente, die im Inneren des Reaktors angebracht werden, herbeigeführt werden kann. Folglich wird die Reaktionsmasse innerhalb des Strömungsrohrs in eine Art schraubenförmige Bewegung versetzt und zusätzlich vom äußeren Rand des Fermenters zur Mittelachse des Fermenters gelenkt. Dies fördert den radialen Temperaturausgleich im Inneren des Fermenters und unterstützt das gewünschte axiale Temperaturgefälle.

Zur Steuerung der natürlichen Zersetzungsprozesse ist eine Temperiervorrichtung vorgesehen, die in axialer Richtung des Fermenters die Segmente streckenweise gezielt erwärmen oder kühlen kann. Damit können im kontinuierlich verlaufenden Prozess im Fermenter - u.a. in Abhängigkeit der Zusammensetzung des Substrates sowie von der Außentemperatur - die mikrobakteriellen Prozesse streckenweise gezielt gebremst oder beschleunigt werden. Damit kann im Einzelnen u.a. vermieden werden, dass die thermophile mikrobakterielle Phase nicht heiß läuft und dass die mesophile mikrobakterielle Phase in den einzelnen Phasen des kontinuierlichen Ablaufs im Fermenter beschleunigt anläuft. Ein weiterer energetisch wirtschaftlicher Vorteil der Verwendung einer zonenweise arbeitenden Temperiervorrichtung ist, dass die freiwerdende Prozesswärme der natürlichen Zersetzungsprozesse, die durch gezielte und prozesstechnisch erforderliche Kühlung der thermophilen Bereiche im Fermenter gewonnen wird, wiederverwendet werden kann, auch direkt im Prozess, nämlich u.a. für Vorerwärmung der mesophilen Bereiche am Anfang des Fermenters.

Das bakterielle Wachstum innerhalb des Fermenters findet in Abhängigkeit des kontinuierlich ablaufenden Prozesses statt. Es kann vorgesehen sein, dass eine Bakterien-Rückkoppelung durchgeführt wird, jedoch nur gezielt in einzelnen Bereichen des Fermentes. Diese Bakterien-Rückkoppelung ist dadurch gekennzeichnet, dass die Bakterien abhängig von der Temperatur und somit von der Position im Fermenter in andere Zonen oder Segmente zurückgekoppelt werden.

Erfindungsgemäß ist eine temperaturgesteuerte Bakterien-Rückkoppelung vorgesehen. Damit können mesophile Bakterien wieder in mesophile Fermenterbereiche rückgeführt und thermophile Bakterien wieder in thermophile Fermenterbereiche zurückgeführt werden. Erfindungsgemäß ist bevorzugt vorgesehen, dass die Bakterien vor einem statischen Führungsleitblech rückgekoppelt werden.

Die Fermentersegmente, durch die das Substrat strömt, können als Wärmetauscher, z.B. nach dem Gegenstromprinzip betrieben, ausgebildet sein.

Beispielsweise kann der Fermenter eine Länge von 75 m und fünf Segmente aufweisen, die wiederum jeweils 15 m lang sind. Die fünf Segmente stellen verschiedene Prozessstufen dar, die mit verschiedenen Prozesstemperaturen arbeiten können. Im Rahmen einer ersten Prozessstufe, in dem ersten Segment, wird dabei beispielsweise eine mesophile Betriebsweise eingeleitet, indem die Temperatur des Substrats mittels der Wärmetauscherfunktion des Fermentersegments gezielt gesteuert wird. In den darauffolgenden zwei Prozessstufen, d. h. im zweiten und dritten Segment, und ab einer Fermenterlänge von 15 m bis zu einer Fermenterlänge von 45 m wird die Substrattemperatur derart geregelt, dass die thermophile Phase eingeleitet wird. Durch die entstehende Reaktionswärme im Fermenter werden mittels der Wärmetauscher der einzelnen Fermentersegmente und der damit dazugehörigen Prozesstemperatursteuerung des Fermenters gezielt gekühlt.

Das Substrat durchläuft während seiner Beförderung mittels Verdrängung durch den Fermenter vorzugsweise in ungerührter Form, bevorzugt mit statischen strömungsbildenden Elementen, eine mesophile als auch eine thermophile Phase. Aufgrund der unterschiedlichen Prozesse der Hydrolyse und Vergärung, d. h. aufgrund von biochemischen und mikrobakteriellen Prozessen, welche sich u.a. sowohl in unterschiedlichen Prozesstemperaturen als auch in unterschiedlichen pH-Werten widerspiegeln, wird die Hydrolyse-Phase vorab getrennt durchgeführt. Des Weiteren würde ohne vorgeschaltete Hydrolyse-Phase die mikrobakterielle Umsetzung der Substratkomponente Holz aufgrund ihrer Strukturen der langen Zelluloseketten sehr viel Zeit in Anspruch nehmen.

Im Rahmen der Erfindung kann somit Holz als Substratkomponente wirtschaftlich verarbeitet werden. Im Rahmen der Erfindung ist es zudem möglich, freiwerdende Prozesswärme der Vergärung weiter zu nutzen bzw. einer weiteren Nutzung zuzuführen, entweder beispielsweise im Umsetzungsprozess der Vergärung selbst oder in der dem erfindungsmäßigen Verfahren vorgeschalteten Hydrolyse.

Bei einer bevorzugten Ausführungsform des erfindungsgemäßen Verfahrens durchströmt das Substrat ein Strömungsrohr, das in Segmente mit unterschiedlichen Längen und/oder mit unterschiedlichen Durchmessern unterteilt ist. Über unterschiedliche Längen können unterschiedliche Verweilzeiten in einer bestimmten Temperaturzone und damit unterschiedliche Reaktionszeiten eingestellt werden. Über den Außendurchmesser eines Segments kann die lokale Abkühlrate beeinflusst werden, insbesondere wenn eine Temperierung über eine Beregnung des Außenmantels des Segments vorgesehen ist. Änderungen der Segmentdurchmesser können also Auswirkungen auf die Prozesstemperatur haben und somit ebenfalls sowohl einen mesophilen als auch einen thermophilen Prozess innerhalb des Fermenters einleiten.

Bei einer weiteren Ausführungsform des erfindungsgemäßen Verfahrens durchströmt das Substrat Segmente mit identischer Länge und identischem Querschnitt oder Segmente mit identischer Länge und unterschiedlichem Querschnitt oder Durchmesser.

Insbesondere können also die Fermenter in Richtung des Substratauslasses einen größer werdenden Querschnitt aufweisen und beispielsweise eine konische, pyramidische oder zylindrische Form annehmen.

Bevorzugt laufen in dem Fermenter ausschließlich die Stufen der Acetogenese, Acidogenese sowie Methanogenese ab. Durch eine weitgehende Auslagerung der Hydrolyse wird erreicht, dass in dem Fermenter eine mesophile und eine thermophile Umsetzung optimiert werden können. Bei der Hydrolyse handelt es sich um eine (bio-)chemische Reaktion, die gemäß der Arrhenius-Gleichung beschrieben werden kann, wonach mit einer steigenden Temperatur eine Erhöhung der Reaktionsgeschwindigkeit einhergeht. Eine optimale und zügige Hydrolyse läuft bei Temperaturen von z.B. 105°C weit oberhalb mesophiler und thermophiler Prozesse ab. Eine thermophile bakterielle Umsetzung findet unterhalb und bis zu einem Temperaturbereich von maximal 55°C statt.

Das erfindungsgemäße Verfahren lässt sich somit besonders bevorzugt im Rahmen einer mehrstufigen Anlage einsetzen, in der die Hydrolyse-Phase in einem getrennten Verfahren stattfindet und dem erfindungsgemäßen Verfahren vorgelagert ist.

Auch die Reaktionsbedingungen der Phasen weichen erheblich voneinander ab; die Hydrolyse-Phase findet bevorzugt in einer leicht säuerlichen Umgebung statt, beispielsweise bei einem pH-Wert 4 - 5; der bakteriellen Vergärungsprozess findet bevorzugt unter leicht alkalischen Bedingungen (pH 7 - 8) statt. Auch vor diesem Hintergrund ist es sinnvoll, die Prozesse der Hydrolyse und mikrobakterielle Vergärung räumlich voneinander abzukoppeln. Eine Rest-Hydrolyse des Substrats wird jedoch auch im erfindungsgemäßen Fermenter stattfinden, diese ist jedoch unerheblich.

Eine Vorrichtung zur Durchführung des Verfahrens ist Gegenstand von Anspruch 9, wobei die Vorrichtung einen Fermenter aufweist, der abschnittsweise temperierbar ist und insbesondere in Segmente unterteilt ist, die streckenweise temperiert werden können bzw. denen jeweils ein Wärmetauscher zugeordnet ist. Die Segmente können dabei beispielsweise als doppelwandiges Rohr ausgeführt sein, wobei sich in dem Raum zwischen den Wänden ein weiteres Medium für den Wärmeaustausch befinden kann.

Die erfindungsgemäße Vorrichtung verzichtet auf mechanische Rührwerke, Schneckentransportvorrichtungen oder Paddelrührwerke im Inneren des Fermenters. Gemäß dem Vorsatz die natürlichen Zersetzungsprozesse nachzuempfinden, befinden sich im inneren des Fermenters keine mechanisch angetriebenen Bauteile.

Eine aktive, prozessgesteuerte Temperiervorrichtung zur streckenweisen Kühlung oder Erwärmung des Fermenterrohres wird vorzugsweise als Besprühungs- oder Beregnungsanlage ausgeführt. Wasser oder eine andere geeignete Flüssigkeit wird von außen auf den Außenmantel des Strömungsrohrs bzw. dessen einzelner Segmente gesprüht.

Um von den Vorteilen eines Pfropfenstromfermenters Gebrauch zu machen, sieht eine praktikable Variante der Erfindung vor, dass der Fermenter als in Segmente geteilten Pfropfenstromfermenter ausgebildet ist, der aufgrund des stabilen Pfropfenstroms biologisch wie mechanisch betriebssicher ist.

Bei den Segmenten des Fermenters kann es sich um einfach miteinander zu verbindende Module handeln, sodass der Fermenter eine modulare Bauform aufweist und somit relativ einfach transportiert und vor Ort zusammengebaut werden kann.

Weitere Varianten und Vorteile der Erfindung werden nachfolgend mit Bezug auf die Zeichnung näher erläutert. Es zeigen jeweils in schematischer Darstellung:
- Fig. 1 - 3: jeweils ein zur Durchführung des Verfahrens geeigneter Reaktionsbehälter im Schnitt;
- Fig. 4: eine grafische Darstellung des Temperaturverlaufs innerhalb und in Längsrichtung des Fermenters;
- Fig. 5: einen Fermenter mit Leitblechen im Längsschnitt und
- Fig. 6: einen Fermenter mit Temperiervorrichtung im Querschnitt.

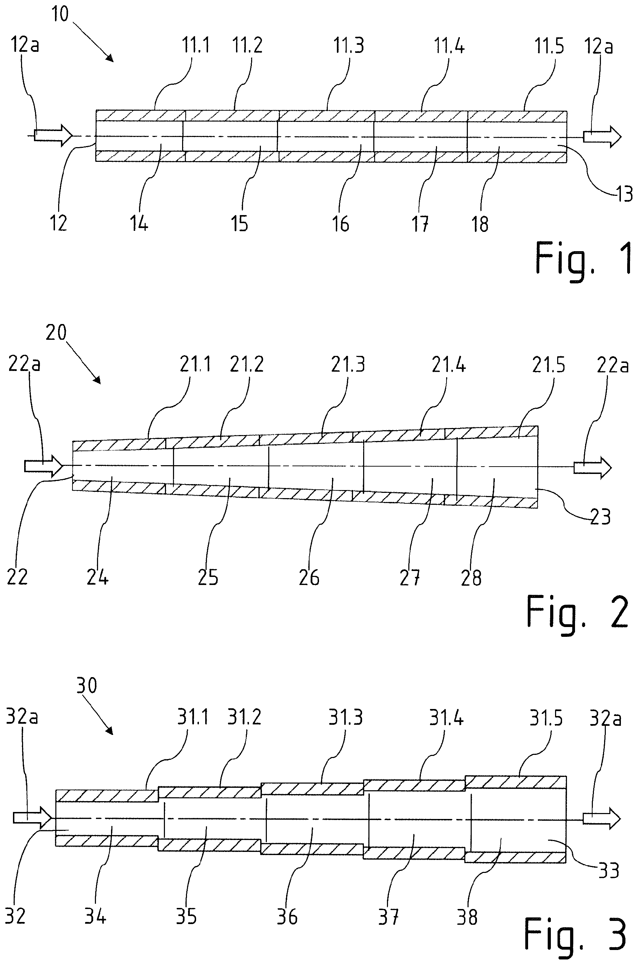

Das erfindungsgemäße Verfahren kann auf Grundlage eines in Fig. 1 in einer seitlichen Schnittdarstellung gezeigten liegenden Fermenters 10 erfolgen, der in der in Fig. 1 dargestellten Ausführungsform in Gestalt eines Pfropfenstromfermenters vorliegt.

Der Fermenter 10 weist eine Länge von 75 m auf und besteht aus einem Strömungsrohr, das in mehrere Segmente 14, 15, 16, 17, 18 unterteilt ist, die unmittelbar hintereinander angeordnet sind. Die Länge jedes einzelnen Segments 14, 15, 16, 17, 18 beläuft sich auf 15 m. Bei der in Fig. 1 dargestellten Ausführungsform des Fermenters 10 liegen die Segmente 14, 15, 16, 17 ,18 in Gestalt von lösbar miteinander verbundenen Modulen vor, sodass der Fermenter 10 in die Segmente 14, 15, 16, 17, 18 zerlegt werden kann.

Jedem Segment 14, 15, 16, 17, 18 ist durch die doppelwandige Konstruktion der Segmente 14, 15, 16, 17, 18 jeweils ein Wärmeaustauscher 11.1, 11.2, 11.3, 11.4, 11.5 mit einer Wärmetauscherfunktion zugeordnet. Zwischen der inneren und äußeren Segmentwandung befindet sich das Wärmetauschermedium mit einer Wärmetauschermediumtemperatur. Bei der in Fig. 1 gezeigten Ausführungsform des Verfahrens wird im herkömmlichen Gegenstromverfahren gearbeitet.

Das Verfahren beginnt damit, dass eine Beschickung des Fermenters 10 erfolgt und zwar derart, dass Substrat an dem Einlass 12 des Fermenters 10 in herkömmlicher Weise in das Innere des Fermenters 10 gegeben wird. Aufgrund des relativ hohen Feststoffgehaltes des Substrats, z. B. 20 - 30 %, durchfließt das Substrat in Strömungsrichtung 12a den Fermenter 10 in Form einer relativ stabilen Masse gemäß dem Pfropfenstromverfahren und wird am Auslass 13 des Fermenters 10 als behandeltes Substrat in herkömmlicher Weise aus dem Fermenter 10 geführt. Die Beförderung des Substrats in dem Fermenter 10 geschieht durch Verdrängung mittels Eingabe des Frischmaterials in den Fermenter 10. Das Substrat in dem Fermenter 10 bleibt im Wesentlichen in ungerührtem Zustand. Statisch strömungsbildende Elemente in dem Fermenter 10 können den Substrattransport führen.

Zuvor hat das Substrat die einzelnen Segmente 14, 15, 16, 17, 18 in Strömungsrichtung 12a passiert und dabei drei Stufen der mikrobiellen Produktion von Biogas, d. h. die Acidogenese, die Acetogenese, sowie die Methanogenese durchlaufen. Das hierbei produzierte Biogas sammelt sich im oberen Bereich der einzelnen Segmente 14, 15, 16, 17, 18 und wird punktuell aus jedem Segment 14, 15, 16, 17, 18 oder am Ende des Segmentes 18 am Auslass 13 aus dem Fermenter 10 abgeführt.

Ein Verfahren zur Herstellung von Biogas mit einem geeigneten Fermenter ist in einem mehrstufigen Prozess eingebunden, d. h. die erste Stufe des Biogaserzeugungsprozesses, die Hydrolyse, findet in dem Fermenter 10 nicht oder nur noch zu einem geringen Teil als Rest-Hydrolyse statt. Bei dem in den Fermenter 10 eingegebenen Substrat handelt es sich damit zum überwiegenden Teil um hydrolysiertes Substrat. Somit stellt der in Fig. 1 gezeigte Fermenter 10 einen Fermenter dar, der der Hydrolyse als rein chemischer oder enzymatischer sowie biochemischer Prozess räumlich nachgeschaltet ist und in dem zum übergroßen Teil nur die bakteriellen Umsetzungsprozesse der Acidogenese, der Acetogenese und der Methanogenese stattfinden, deren Geschwindigkeitsablauf an Aktivität und Wachstumsrate der entsprechenden Bakterien gekoppelt sind. Bezogen auf die mikrobakterielle Produktion von Biogas bedeutet dies, dass die meisten mesophilen Bakterienarten ihre maximale Aktivität und Wachstumsgeschwindigkeit in einem Bereich zwischen 25°C und 40°C erreichen. Die Reaktionsgeschwindigkeit thermophiler Bakterienarten setzt wiederum erst bei Temperaturen über 40°C ein. Um diesen gewünschten und erforderlichen Prozesstemperaturen Genüge zu leisten, wird das in den Fermenter 10 eingebrachte Substrat in den einzelnen Segmenten 14, 15, 16, 17, 18 während des Durchströmens des Substrats in Strömungsrichtung 12a durch die Segmente 14, 15, 16, 17 ,18 mittels gezielter Kühlung oder Erwärmung auf mesophile und thermophile Prozesstemperaturen gebracht. Hierbei geht die in dem Fermenter 10 einsetzende Vergärung mit der Erzeugung von Reaktionswärme einher.

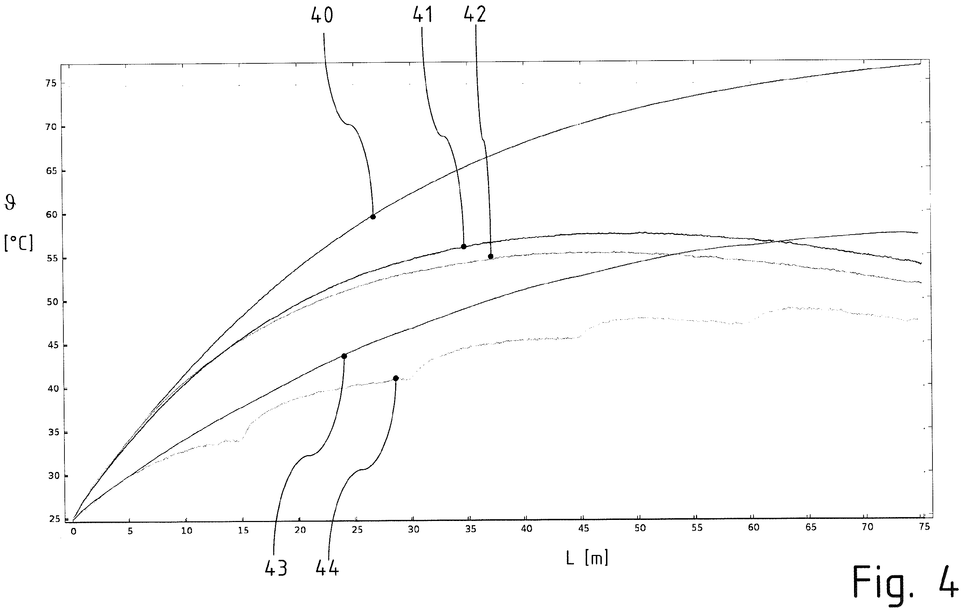

Der Verlauf der Reaktionswärme geht aus der grafischen Darstellung in Fig. 4 hervor. Das Substrat beinhaltet rund 1/3 hydrolysiertes Holz sowie Grünabfälle, Mist und Gülle. Speziell Holz mit seinen langkettigen Cellulose-Strukturen muss hauptsächlich vor der Vergärung in einem separaten vorgelagerten Prozess hydrolysiert werden.

Fig. 4 zeigt die Temperaturentwicklung des in den Fermenter 10 eingebrachten Gärsubstrats in Abhängigkeit von der Länge des Fermenters 10. Wie bereits beschrieben, weist der in Fig. 1 gezeigte Fermenter 10 eine Länge von 75 m und die Segmente 14, 15, 16, 17, 18 jeweils eine Länge von 15 m auf. Die Gesamtdurchlaufzeit beträgt im vorliegenden Fall ca. 30 Tage, d. h. eine Substrat-Durchlaufgeschwindigkeit von rund 2,5 m/Tag. Ohne zusätzliche Temperatursteuerung des anaeroben Vergärungsprozesses in dem Fermenter 10, d. h. Herstellung mesophiler oder thermophiler Prozessabläufe mittels Kühlung, würde das frisch eingebrachte und aufgeheizte Substrat in dem Fermenter 10, wie der Temperaturgraph 40 in Fig. 4 verdeutlicht, infolge von freigesetzter Prozesswärme schon nach relativ kurzer Verweilzeit von rd. 10 - 12 Tagen des Substrats in dem Fermenter 10, d. h. nach 25 - 30 m, eine Temperatursteigung von 25°C auf ca. 60°C und am Ende des Fermenters 10, d. h. nach ca. 75 m, gar eine Temperatur von theoretisch über 75°C aufweisen. In der Praxis würde der Prozess aber lange vorher beim Verlassen des thermophilen Bereichs bei ca. 50 - 55°C verlangsamen und letztendlich komplett zum Erliegen kommen.

Um während des Durchströmens von Substrat in Strömungsrichtung 12a durch den Fermenter 10 mesophile und thermophile Prozesstemperaturen zu gewährleisten, wird schrittweise in den Segmenten 14, 15, 16, 17, 18 mittels den Wärmetauscher 11.1, 11.2, 11.3, 11.4, 11.5 in den einzelnen Fermentersegmente 14, 15, 16, 17, 18 das durch den Fermenter 10 durchströmende Substrat abgekühlt. Im Rahmen einer ersten Prozessstufe in dem Fermenter 10 findet dabei eine Kühlung auf einer Strecke von 0 bis 15 m statt. In dieser Stufe wird das eingebrachte Substrat mit einer Wärmetauschermediumtemperatur von z.B. 20°C gekühlt. Wie der Temperaturgraph 41 in Fig. 4 verdeutlicht, findet in diesem Bereich im ersten Segment zwischen 0 und 15 m eine Vergärung des Substrats in dem Fermenter 10 bei mesophilen Prozesstemperaturen statt. In den darauffolgenden zwei Segmenten 15 und 16, auf einer Strecke zwischen 15 m und 45 m, steigt die Prozesstemperatur des Substrats trotz Kühlung relativ schnell auf einem Bereich von rd. 55°C. Dabei wird in vorliegendem Beispiel in Segment 15 mit einer Wärmetauschermediumtemperatur von 22,5°C und in Segment 16 mit einer Wärmetauschermediumtemperatur von 25°C gekühlt.

Der Temperaturgraph 41 in Fig. 4 zeigt den Temperaturverlauf des Substrats in Strömungsrichtung 12a in der Mitte der Längsachse des Fermenters 10. Der Temperaturgraph 42 in Fig. 40 zeigt den Temperaturverlauf des Substrats in Strömungsrichtung 12a, und zwar in einer Distanz von 0,5 m von der Mitte der Längsachse des Fermenters 10 entfernt.

Aus energetischer Sicht ist es wünschenswert, dass das durch den Fermenter 10 durchströmende Substrat den Fermenter 10 am Auslass 13 mit einer möglichst geringen Temperatur verlässt. Die hierbei freiwerdende Energie, erzeugt aus der Reaktionswärme, kann z.B. für die räumlich und zeitlich vorgeschaltete Hydrolyse genutzt werden. Aus diesem Grund werden die letzten beiden Segmente 17 und 18, wieder mit niedrigerem Wärmetauschermediumtemperatur gekühlt, z.B. mit 22,5°C für Segment 17 und 20°C für Segment 18. Wie der Temperaturgraph 41 und 42 in Fig. 4 zeigen, hat diese Vorgehensweise zur Folge, dass die Substrat-temperatur in den Segmenten 17 und 18, d. h. auf der Strecke zwischen 45 m und 75 m sinkt. Der Vergleich zwischen den Temperaturgraphen 41 und 42 zeigt zudem, dass es in radialer Richtung in dem Fermenter ein Temperaturgefälle gibt, bedingt auch durch die ungerührte Form des Substrats. Das radiale Temperaturgefälle in dem Fermenter 10 fördert die partielle Bildung von Bakterienkulturen und unterstützt damit die Übergänge der einzelnen mesophilen und thermophilen Prozessphasen und fördert damit die entsprechende Vergärung des Substrats.

Die Fig. 2 und 3 zeigen weitere geometrische Ausführungsformen eines für das erfindungsgemäße Verfahren einsetzbaren Pfropfenstromfermenters 10.

Die in den Fig. 2 und 3 gezeigten Fermenter, die mit den Bezugszeichen 20 und 30 versehen sind, weisen ebenfalls Segmente 24, 25, 26, 27, 28 und 34, 35, 36, 37, 38 auf, denen wiederum jeweils Temperiervorrichtungen wie z.B. Wärmetauscher 21.1, 21.2, 21.3, 21.4, 21.5 und 31.1, 31.2, 31.3, 31.4, 31.5 mittels doppelwandiger Konstruktion der Segmente 24, 25, 26, 27, 28 und 34, 35, 36, 37, 38 zugeordnet sind. An den beiden Enden der Fermenter 20 und 30 befinden sich zudem ebenfalls Ein- und Auslässe, die mit den Bezugszeichen 22, 23 und 32, 33 gekennzeichnet sind. An den Einlässen 22 und 32 wird Substrat in Strömungsrichtung 22a und 32a den Fermentern 20 und 30 zugeführt und an den Auslässen 23 und 33 das in den Fermentern 20 und 30 behandelte Substrat wieder abgeführt.

Im Gegensatz zu der in Fig. 1 gezeigten Ausführungsform des Fermenters 10 steigt der Durchmesser der Segmente 24, 25, 26, 27, 28 des Fermenters 20 und der Segmente 34, 35, 36, 37, 38 des Fermenters 30 in Richtung der Auslässe 23 und 33 an. Ein steigender Querschnitt oder Durchmesser von 1,5 m am Anfang des ersten Segments bis auf 2,5 m am Ende des letzten Segments erweisen sich als vorteilhaft für die Prozesstemperatursteuerung und den allgemeinen Prozessablauf. Bei einem gestuften Verlauf statt eines konischen oder pyramidischen Verlaufs der Geometrie des Fermenters 30 erweisen sich auf der Grundlage der Analysen und Simulationen Durchmesseränderungen von 0,25 m als zweckmäßig für einen wirtschaftlichen und reibungslosen Prozessablauf.

Die nächsten Temperaturgraphen 43 und 44 gehen von Segmentdurchmessern von sukzessive 1,5m, 1,75m, 2,0m 2,25m sowie 2,5m aus, wie sie in dem Fermenter 30 in Fig. 3 umgesetzt sind.

Die geometrischen Gestaltungen des Fermenters 30 haben, wie die Temperaturgraphen 43 und 44 in Fig. 4 zeigen, Auswirkungen auf den Verlauf der Prozesstemperaturen in den einzelnen Segmenten 34, 35, 36, 37 und 38 des Fermenters 30. Es werden stets durch entsprechende Kühlungen mittels der Wärmetauscher 31.1, 31.2, 31.3, 31.4 und 31.5 der doppelwandigen Segmente 34, 35, 36, 37 und 38 des Fermenters 30 mesophile und thermophile Prozessstufen erreicht, wobei diese Prozesstemperaturen insbesondere in der ersten Hälfte des Fermenters 30 niedriger als bei dem in Fig. 1 gezeigten Fermenter 10 sind. Wie aus den Temperaturgraphen 43 und 44 außerdem hervorgeht, wird das radiale Temperaturgefälle des Substrats in Fermenter 30 größer als in Fermenter 10.

Der Temperaturgraph 43 in Fig. 4 zeigt den Temperaturverlauf des Substrats in Strömungsrichtung 32a in der Mitte der Längsachse des Fermenters 30.

Der Temperaturgraph 44 in Fig. 4 zeigt den Temperaturverlauf des Substrats in Strömungsrichtung 32a in einer Distanz von 0,5 m am Anfang des Fermenters 30 bis 0,75 m am Ende des Fermenters 30 von der Mitte der Längsachse des Fermenters 30 aus gesehen.

Vielmehr ist eine Vielzahl von Varianten denkbar, welche von der dargestellten Lösung auch bei grundsätzlich anders gearteten Ausführungen Gebrauch machen, sofern sie vom Schutzumfang der Ansprüche abgedeckt sind. Beispielsweise können während der Behandlung des Substrats in Fermentern mit geänderten Geometrien gemäß unabhängigem Anspruch 9 mittels beschriebenen sequentiellen Wärmetauscherfunktionen und einer entsprechenden dynamischen Prozesstemperatursteuerung weitere unterschiedliche Temperaturverläufe, die mesophile und thermophile Verfahrensabläufe erfüllen, erzeugt werden.

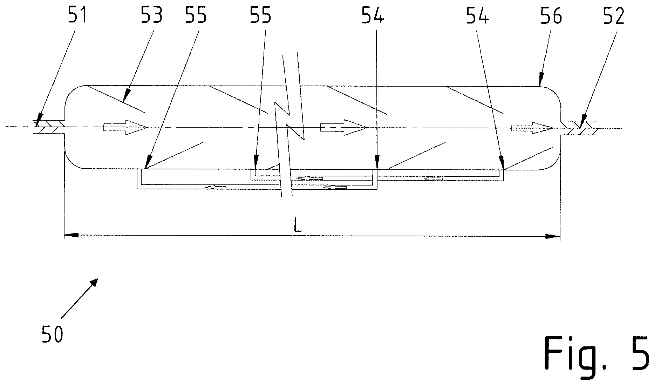

In Figur 5 ist ein Längsschnitt eines Fermenters 56 ohne Temperiervorrichtung dargestellt, der sich zwischen einem Fermentereinlass 51 und einem Fermenterauslass 52 erstreckt.

Die temperaturgesteuerten Bakterien-Ein- und-Auslassleitungen 54, 55 können in Abhängigkeit von der Substratzusammensetzung und der Länge L des Fermenters 56 beliebig oft angebracht werden,

Ein Führungsleitblech 53 versetzt die Reaktionsmasse in eine axiale und rotierende Bewegung und lenkt diese zusätzlich vom Rand des Fermenters zur Mittelachse des Fermenters.

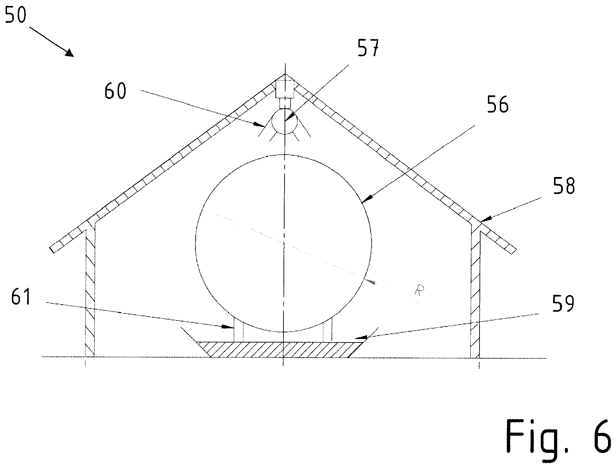

In Figur 6 ist der Fermenter 56 im Querschnitt sowie die bei der bevorzugten Ausführungsform vorgesehene Temperiervorrichtung dargestellt. Die Temperiervorrichtung erfolgt bei dieser Ausführungsform nicht in Form eines in die Wandung des Strömungsrohrs integrierten Wärmetauschers, sondern der gesamte Fermenter wird unter einer Überdachung 58 platziert. Oberhalb des Strömungsrohrs 56 befindet sich eine Sprühvorrichtung 57, die z.B. als Rohr-Beregnungsanlage gestaltet ist. Das Strömungsrohr 56 ist über Befestigungsmittel 61 oberhalb eines Auffangbeckens 59 aufgeständert.

Mithilfe der Sprühvorrichtung 57 und des Auffangbeckens 59 für das Temperierwasser 60 sowie eines nicht näher dargestellten Pumpenkreislaufs kann der Fermenter kontinuierlich temperiert werden, indem einfach seine Wandung von oben mit mehr oder weniger Temperierwasser 60 benetzt wird. Mittels einer Aufteilung der Sprühvorrichtung 57 in einzelne Bereiche in axialer Richtung kann die Temperierung gezielt in entsprechenden Segmenten des Fermenters 56 durchgeführt werden. Damit können die einzelnen mikrobakteriellen Phasen in dem kontinuierlich verlaufenden Prozess gezielt temperiert werden.

### Bezugszeichenliste:

- 10: Fermenter
- 11.1 bis 11.5: Wärmetauscher
- 12: Einlass
- 12a: Strömungsrichtung
- 13: Auslass
- 14 bis 18: Segmente

- 20: Fermenter
- 21.1 bis 21.5: Wärmetauscher
- 22: Einlass
- 22a: Strömungsrichtung
- 23: Auslass
- 24 bis 28: Segmente

- 30: Fermenter
- 31.1 bis 31.5: Wärmetauscher
- 32: Einlass
- 32a: Strömungsrichtung
- 33: Auslass
- 34 bis 38: Segmente

- 40 bis 44: Temperaturgraphen

- 50: Fermenter
- 51: Fermentereinlass
- 52: Fermenterauslass
- 53: Führungsleitblech
- 54: Einlass temperaturgesteuerte Bakterien-Rückkoppelung
- 5: Auslass temperaturgesteuerte Bakterien-Rückkoppelung
- 56: Fermenter
- 57: Sprühanlage
- 58: Überdachung
- 59: Temperiermedium-Auffangbecken
- 60: Austretendes Temperiermedium
- 61: Befestigungselemente

## Patentansprüche

1. Verfahren zur Herstellung von Biogas in einem Fermenter (10, 20, 30; 50),
- wobei an einem Ende des Fermenters (10, 20, 30, 50) das Substrat eingegeben und am anderen Ende des Fermenters (10, 20, 30, 50) das behandelte Substrat abgezogen wird,
- wobei der Fermenter (10, 20, 30, 50) wenigstens ein einzelnes Strömungsrohr umfasst, das zwischen den beiden Enden des Strömungsrohrs in Strömungsrichtung (12a, 22a, 32a) von dem Substrat durchströmt wird,
**dadurch gekennzeichnet,**
- **dass** das Substrat durch ein liegendes Strömungsrohr geleitet wird, das zonenweise unterschiedlich temperiert wird.
- **dass** das Substrat zwischen Eingang und Ausgang des Fermenters (10, 20, 30, 50) mehrere Zersetzungsphasen durchläuft und während des Durchlaufs durch den Fermenter (10, 20, 30, 50) sowohl in einem mesophilen Temperaturbereich als auch in einen thermophilen Temperaturbereich gebracht wird und
- **dass** das Substrat kontinuierlich mittels Verdrängung als Pfropfenstrom durch den Fermenter (10, 20, 30, 50) strömt, wobei das Substrat ungerührt, ohne motorisch angetriebene Mischer, durch den Fermenter (10, 20, 30, 50) geführt wird.

2. Verfahren nach Anspruch 1, **dadurch gekennzeichnet, dass** das Strömungsrohr in hintereinander angeordnete Segmente (14 bis 18, 24 bis 28, 34 bis 38) geteilt ist, in denen das Substrat unterschiedlich temperiert wird.

3. Verfahren nach Anspruch 2, **dadurch gekennzeichnet, dass** die Segmente (14 bis 18, 24 bis 28, 34 bis 38) jeweils als Wärmetauscher (11.1 bis 11.5, 21.1 bis 21.5, 31.1 bis 31.5) ausgebildet sind, über die das Substrat gekühlt oder erwärmt wird.

4. Verfahren nach einem der Ansprüche 1 bis 3, **dadurch gekennzeichnet, dass** das Substrat durch Segmente (14 bis 18, 24 bis 28, 34 bis 38) mit unterschiedlichen Längen und/oder mit unterschiedlichen Durchmessern strömt.

5. Verfahren nach einem der vorhergehenden Ansprüche, **dadurch gekennzeichnet, dass** die Reaktionsmasse innerhalb des Strömungsrohrs in eine schraubenförmige Bewegung versetzt wird.

6. Verfahren nach einem der vorhergehenden Ansprüche, **dadurch gekennzeichnet, dass** Bakterien abhängig von der Temperatur und somit von der Position im Fermenter in andere Zonen oder Segmente zurückgekoppelt werden.

7. Verfahren nach einem der vorhergehenden Ansprüche, **dadurch gekennzeichnet, dass** das Substrat vor der Einleitung in den Fermenter (10, 20, 30, 50) hydrolysiert wird.

8. Verfahren nach einem der vorhergehenden Ansprüche, **dadurch gekennzeichnet, dass** das Substrat im Fermenter (10, 20, 30, 50) durch Besprühung der Außenhaut des Strömungsrohrs mit einer Flüssigkeit temperiert wird.

9. Vorrichtung zur Durchführung des Verfahrens gemäß einem der Ansprüche 1 bis 8, **gekennzeichnet durch** einen Fermenter, der genau ein liegendes Strömungsrohr (10, 20, 30, 50) umfasst, das keine angetriebene mechanische Rühreinrichtung aufweist und das in Strömungsrichtung (12a, 22a, 32a) in Temperierzonen oder in Segmente (14 bis 18, 24 bis 28, 34 bis 38) unterteilt ist, welche einzeln oder gruppenweise unterschiedlich temperierbar sind und sowohl in einem mesophilen Temperaturbereich als auch in einem thermophilen Temperaturbereich gebracht werden können.

10. Vorrichtung (20) nach Anspruch 9, **dadurch gekennzeichnet, dass** die Segmente (24 bis 28) jeweils unterschiedliche und in Strömungsrichtung (22a) größer werdende Durchmesser aufweisen.

11. Vorrichtung nach Anspruch 9, **dadurch gekennzeichnet, dass** sich die Segmente (34 bis 38) jeweils konisch in Strömungsrichtung (32a) erweitern und zusammengesetzt ein konisches Strömungsrohr (30) bilden.

12. Vorrichtung nach einem der Ansprüche 9 bis 11, **dadurch gekennzeichnet, dass** die Segmente (14 bis 18, 24 bis 28, 34 bis 38) jeweils unterschiedliche Längen aufweisen.

13. Vorrichtung (10) nach einem der Ansprüche 9 bis 12, **dadurch gekennzeichnet, dass** den Segmenten (14 bis 18, 24 bis 28, 34 bis 38) jeweils eine Temperiervorrichtung (11.1 bis 11.5, 21.1 bis 21.5, 31.1 bis 31.5, 57) zugeordnet ist.

14. Vorrichtung nach einem der Ansprüche 9 bis 13, **dadurch gekennzeichnet, dass** die Segmente (14 bis 18, 24 bis 28, 34 bis 38) als lösbar miteinander verbundene Module vorliegen.

15. Vorrichtung nach einem der Ansprüche 9 bis 14, **dadurch gekennzeichnet, dass** im Inneren des Strömungsrohrs des Fermenters (50) statische, strömungsleitende Elemente (53) angeordnet sind.

## Claims

1. Process of producing biogas in a fermenter (10, 20, 30, 50),
- wherein the substrate is introduced at one end of the fermenter (10, 20, 30, 50) and the treated substrate is drawn off at the other end of the fermenter (10, 20, 30, 50),
- wherein the fermenter (10, 20, 30, 50) comprises at least one single flow tube through which the substrate flows in flow direction (12a, 22a, 32a) between the two ends of the flow tube,
**characterized in that**
- the substrate is guided through a horizontal flow tube having zones heated to different temperatures,
- the substrate undergoes multiple breakdown phases between the inlet and outlet of the fermenter (10, 20, 30, 50) and, while passing through the fermenter (10, 20, 30, 50), is brought both into a mesophilic temperature range and into a thermophilic temperature range, and
- the substrate flows continuously through the fermenter (10, 20, 30, 50) in the form of a plug flow by means of displacement, wherein the substrate is guided through the fermenter (10, 20, 30, 50) without stirring and without a motordriven mixer.

2. Process according to Claim 1, **characterized in that** the flow tube is divided into sequentially arranged segments (14 to 18, 24 to 28, 34 to 38) in which the substrate is heated to different temperatures.

3. Process according to Claim 2, **characterized in that** the segments (14 to 18, 24 to 28, 34 to 38) each take the form of a heat exchanger (11.1 to 11.5, 21.1 to 21.5, 31.1 to 31.5) by means of which the substrate is cooled or heated.

4. Process according to any of Claims 1 to 3, **characterized in that** the substrate flows through segments (14 to 18, 24 to 28, 34 to 38) having different lengths and/or having different diameters.

5. Process according to any of the preceding claims, **characterized in that** the reaction mass is set in a helical motion within the flow tube.

6. Process according to any of the preceding claims, **characterized in that** bacteria are fed back into other zones or segments depending on the temperature and hence on the position in the fermenter.

7. Process according to any of the preceding claims, **characterized in that** the substrate is hydrolysed before being introduced into the fermenter (10, 20, 30, 50) .

8. Process according to any of the preceding claims, **characterized in that** the temperature of the substrate in the fermenter (10, 20, 30, 50) is controlled by spraying the outer skin of the flow tube with a liquid.

9. Apparatus for performing the process according to any of Claims 1 to 8, **characterized by** a fermenter comprising exactly one horizontal flow tube (10, 20, 30, 50) which has no driven mechanical stirrer unit and is divided in flow direction (12a, 22a, 32a) into temperature zones or into segments (14 to 18, 24 to 28, 34 to 38) that can be set to different temperatures individually or in groups, and can be brought both into a mesophilic temperature range and into a thermophilic temperature range.

10. Apparatus (20) according to Claim 9, **characterized in that** the segments (24 to 28) are each of different diameter increasing in flow direction (22a).

11. Apparatus according to Claim 9, **characterized in that** the segments (34 to 38) each extend conically in flow direction (32a) and together form a conical flow tube (30) .

12. Apparatus according to any of Claims 9 to 11, **characterized in that** the segments (14 to 18, 24 to 28, 34 to 38) each have different lengths.

13. Apparatus (10) according to any of Claims 9 to 12, **characterized in that** the segments (14 to 18, 24 to 28, 34 to 38) are each assigned a temperature control device (11.1 to 11.5, 21.1 to 21.5, 31.1 to 31.5, 57) .

14. Apparatus according to any of Claims 9 to 13, **characterized in that** the segments (14 to 18, 24 to 28, 34 to 38) are in the form of modules releasably connected to one another.

15. Apparatus according to any of Claims 9 to 14, **characterized in that** static flow-guiding elements (53) are disposed within the flow tube of the fermenter (50).

## Revendications

1. Procédé de fabrication de biogaz dans un fermentateur (10, 20, 30 ; 50),
- dans lequel le substrat est introduit à une extrémité du fermentateur (10, 20, 30, 50) et le substrat traité est soutiré à l'autre extrémité du fermentateur (10, 20, 30, 50),
- dans lequel le fermentateur (10, 20, 30, 50) comprend au moins un tube d'écoulement individuel, qui est traversé par le substrat entre les deux extrémités du tube d'écoulement dans la direction d'écoulement (12a, 22a, 32a),
**caractérisé en ce que**
- le substrat est conduit au travers d'un tube d'écoulement horizontal, qui est différemment conditionné en température en zones,
- le substrat passe par plusieurs phases de décomposition entre l'entrée et la sortie du fermentateur (10, 20, 30, 50) et est amené aussi bien dans une plage de température mésophile que dans une plage de température thermophile pendant le passage au travers du fermentateur (10, 20, 30, 50), et
- le substrat s'écoule en continu par refoulement en tant qu'écoulement piston au travers du fermentateur (10, 20, 30, 50), le substrat étant acheminé au travers du fermentateur (10, 20, 30, 50) sous forme non agitée, sans mélangeur entraîné par un moteur.

2. Procédé selon la revendication 1, **caractérisé en ce que** le tube d'écoulement est divisé en segments agencés les uns après les autres (14 à 18, 24 à 28, 34 à 38), dans lesquels le substrat est différemment conditionné en température.

3. Procédé selon la revendication 2, **caractérisé en ce que** les segments (14 à 18, 24 à 28, 34 à 38) sont chacun configurés sous la forme d'échangeurs de chaleur (11.1 à 11.5, 21.1 à 21.5, 31.1 à 31.5), par l'intermédiaire desquels le substrat est refroidi ou réchauffé.

4. Procédé selon l'une quelconque des revendications 1 à 3, **caractérisé en ce que** le substrat s'écoule au travers de segments (14 à 18, 24 à 28, 34 à 38) ayant différentes longueurs et/ou ayant différents diamètres.

5. Procédé selon l'une quelconque des revendications précédentes, **caractérisé en ce que** la masse réactionnelle est déplacée à l'intérieur du tube d'écoulement en un mouvement hélicoïdal.

6. Procédé selon l'une quelconque des revendications précédentes, **caractérisé en ce que** des bactéries sont réinjectées dans d'autres zones ou segments en fonction de la température et par conséquent de la position dans le fermentateur.

7. Procédé selon l'une quelconque des revendications précédentes, **caractérisé en ce que** le substrat est hydrolysé avant l'introduction dans le fermentateur (10, 20, 30, 50).

8. Procédé selon l'une quelconque des revendications précédentes, **caractérisé en ce que** le substrat est conditionné en température dans le fermentateur (10, 20, 30, 50) par pulvérisation d'un liquide sur le revêtement extérieur du tube d'écoulement.

9. Dispositif pour la réalisation du procédé selon l'une quelconque des revendications 1 à 8, **caractérisé par** un fermentateur, qui comprend exactement un tube d'écoulement horizontal (10, 20, 30, 50), qui ne comprend pas d'appareil d'agitation mécanique entraîné et qui est divisé dans la direction d'écoulement (12a, 22a, 32a) en zones de conditionnement en température ou en segments (14 à 18, 24 à 28, 34 à 38), qui peuvent être différemment conditionnés en température individuellement ou en groupes, et peuvent être amenés aussi bien dans une plage de température mésophile que dans une plage de température thermophile.

10. Dispositif (20) selon la revendication 9, **caractérisé en ce que** les segments (24 à 28) présentent chacun des diamètres différents et croissants dans la direction d'écoulement (22a).

11. Dispositif selon la revendication 9, **caractérisé en ce que** les segments (34 à 38) s'élargissent chacun coniquement dans la direction d'écoulement (32a) et forment ensemble un tube d'écoulement conique (30).

12. Dispositif selon l'une quelconque des revendications 9 à 11, **caractérisé en ce que** les segments (14 à 18, 24 à 28, 34 à 38) présentent chacun différentes longueurs.

13. Dispositif (10) selon l'une quelconque des revendications 9 à 12, **caractérisé en ce qu'**un dispositif de conditionnement en température (11.1 à 11.5, 21.1 à 21.5, 31.1 à 31.5, 57) est associé respectivement aux segments (14 à 18, 24 à 28, 34 à 38) .

14. Dispositif selon l'une quelconque des revendications 9 à 13, **caractérisé en ce que** les segments (14 à 18, 24 à 28, 34 à 38) se présentent sous la forme de modules reliés de manière amovible les uns avec les autres.

15. Dispositif selon l'une quelconque des revendications 9 à 14, **caractérisé en ce que** des éléments statiques conduisant l'écoulement (53) sont agencés à l'intérieur du tube d'écoulement du fermentateur (50).
